# EUROPEAN PATENT APPLICATION

(11) **EP 4 249 078 A1**
(43) Date of publication of application: **27.09.2023**
(21) Application number: 23163348.8
(22) Date of filing: 22.03.2023
(51) Int. Cl.: A61Q 11/02, A61Q 19/10, A61K 8/43, A61K 8/34

(54) **SULPHITE-FREE ORAL HYGIENE FORMULATIONS DESIGNED TO COUNTERACT TOOTH PIGMENTATION**

(30) Priority: 23.03.2022 IT 202200005747
(71) Applicant: BMG Pharma S.r.l., 20124 Milano (IT)
(72) Inventor: MASTRODONATO, Marco, 20124 MILANO (IT)
(74) Representative: Bianchetti & Minoja with Trevisan & Cuonzo IPS SRL

(57) **Abstract**

Disclosed are compositions for topical use for oral hygiene comprising chlorhexidine or a salt thereof such as gluconate and an alkyl-resorcinol, in particular 4-hexyl-resorcinol, in combination with suitable excipients. Alkyl-resorcinol effectively counteracts the pigmenting action of chlorhexidine on the tooth enamel.

## Description

The invention relates to sulphite-free compositions comprising chlorhexidine and an alkyl-resorcinol, in particular 4-hexyl-resorcinol, in combination with suitable excipients.

### Prior art

Biguanide antimicrobials or antiseptics such as chlorhexidine, alexidine and polyhexanide possess a broad-spectrum antibacterial action and are widely used in oral hygiene formulations and in the ophthalmic and dermatological fields, for example in the form of mouthwashes, gels, eyedrops and contact lens disinfectant solutions.

Chlorhexidine [1,1'-hexamethylenebis[5-(p-chlorophenyl)biguanide], which is poorly water-soluble, is mainly used in gluconate form. When used as a mouthwash for oral hygiene, especially in the dental field, chlorhexidine causes undesirable staining of the tooth enamel. Due to its cationic properties, chlorhexidine binds to the oral mucosa, the hydroxyapatite of tooth enamel, dental bacterial plaque, salivary proteins and cellular polysaccharides of bacterial origin.

The resulting dark yellow or brownish pigmentation on the surface of the teeth and mucosa, or on resin restorations or dentures, prejudices compliance by users.

The use of chlorhexidine-based mouthwashes is therefore contraindicated for times exceeding a few days, because the tooth discolouration is permanent, and difficult to eliminate except by polishing and bleaching procedures. In an attempt to eliminate this drawback, formulations containing sulphites or other antistaining agents have been proposed. For example, EP 2614812 describes a chlorhexidine mouthwash containing sodium metabisulphite, ascorbic acid and hyaluronic acid. EP 2863863 describes chlorhexidine compositions containing an antistaining agent based on at least two ingredients selected from anionic agents (pyrophosphates, phytates and saccharide derivatives), carbonyl compounds and ethoxylated non-ionic surfactants.

However, the use of sulphites involves drawbacks associated with their tolerability profile and allergenic potential, which contraindicate or strongly limit their use in the pharmaceutical, health-care product and cosmetic industries.

A formulation of biguanide antiseptics, especially chlorhexidine, which is sulphite-free and does not present the problem of tooth pigmentation when used as a mouthwash, would therefore be desirable.

### Description of the invention

It has now been discovered that alkyl-resorcinols in general, and hexyl-resorcinol in particular, effectively counteract the pigmenting action of biguanide antiseptics, especially chlorhexidine, in formulations useful for oral and buccal hygiene, for example in the form of aqueous solutions and mouthwashes.

Hexyl-resorcinol (4-hexyl-resorcinol) has anaesthetic, antiseptic and anthelminthic properties, and is used topically for skin infections and as an antiseptic in tooth implantations and for the treatment of pharyngitis. Dilute solutions of 4-hexyl-resorcinol are used in the fishing industry to counteract blackening of crustaceans when exposed to air (Prawnfresh^{®} Xyrex Corporation).

The object of the present invention is therefore compositions, preferably in the form of sulphite-free aqueous solutions, comprising chlorhexidine and an alkyl-resorcinol, in particular 4-hexyl-resorcinol, in combination with suitable excipients.

The compositions according to the invention contain chlorhexidine or a salt thereof, preferably gluconate, in concentrations ranging between 0.001 and 0.1 wt%, preferably between 0.01 and 0.05 wt%, and 4-hexyl-resorcinol in concentrations ranging between 0.001 and 0.05 wt%, preferably between 0.002 and 0.02 wt%.

In addition to the aqueous solvent base, the excipients comprise co-solvents, in particular propylene glycol; surfactants such as poloxamers and polyethoxylated castor oil; flavourings/sweeteners; salts; pH regulators, colourings, viscosity-controlling and mucoadhesive agents. In addition to chlorhexidine, other active ingredients and antiseptics useful for the desired applications may be present. The formulations in solution according to the invention are advantageously administered as mouthwashes for rinsing after dental procedures or for daily maintenance of oral hygiene. No tooth discolouration has been observed with said formulations, unlike conventional chlorhexidine formulations, even when used once or twice a day for three or four weeks.

The invention is illustrated in detail in the examples below.

### Example 1

| • Ingredient | % |
|---|---|
| • PURIFIED WATER Ph. Eur. | 89.67248 |
| • XYLITOL | 5 |
| • PROPYLENE GLYCOL | 3 |
| • KOLLIPHOR P 407 | 1 |
| • CREMOPHOR RH 40 | 0.7 |
| • MINT FLAVOURING 95448 (LIN003440001) | 0.298542 |
| • LIMONENE | 0.00135 |
| • LINALOOL | 0.000108 |
| • HEXYLRESORCINOL (5% solution in water 51%, propylene glycol 25%, citric acid 15% and sodium chloride 4%) | 0.075 (hexylresorcinol 0.0037) |
| • CHLORHEXIDINE DIGLUCONATE 20% aqueous solution | 0.05 (0.01 of chlorhexidine gluconate) |
| • SODIUM HYDRATE drops P.P.A | 0.00252 |
| • MINT / LIQUORICE FLAVOURING | 0.1 |
| • SODIUM SACCHARINE | 0.1 |
| | 100 |

### Example 2

| Ingredient | % |
|---|---|
| PURIFIED WATER Ph. Eur. | 89.398 |
| XYLITOL | 5 |
| PROPYLENE GLYCOL | 3 |
| KOLLIPHOR P 407 | 1 |
| CREMOPHOR RH 40 | 0.7 |
| MINT FLAVOURING 95448 (LIN003440001) | 0.298542 |
| LIMONENE | 0.00135 |
| LINALOOL | 0.000108 |
| HEXYLRESORCINOL (5% solution in water 51%, propylene glycol 25%, citric acid 15% and sodium chloride 4%) | 0.18 (hexylresorcinol 0.009) |
| CHLORHEXIDINE DIGLUCONATE 20% aqueous solution | 0.12 (0.024 of chlorhexidine gluconate) |
| SODIUM HYDRATE drops P.P.A | 0.102 |
| MINT / LIQUORICE FLAVOURING | 0.1 |
| SODIUM SACCHARINE | 0.1 |
| | 100 |
| SODIUM HYDRATE drops P.P.A | 0.102 |
| MINT / LIQUORICE FLAVOURING | 0.1 |
| SODIUM SACCHARINE | 0.1 |
| | 100 |

### Example 3

| Ingredient | % |
|---|---|
| PURIFIED WATER Ph. Eur. | 89.2898 |
| XYLITOL | 5 |
| PROPYLENE GLYCOL | 3 |
| KOLLIPHOR P 407 | 1 |
| CREMOPHOR RH 40 | 0.7 |
| MINT FLAVOURING 95448 (LIN003440001) | 0.298542 |
| LIMONENE | 0.00135 |
| LINALOOL | 0.000108 |
| HEXYLRESORCINOL (5% solution in water 51%, propylene glycol 25%, citric acid 15% and sodium chloride 4%) | 0.3 (hexylresorcinol 0.015) |
| CHLORHEXIDINE DIGLUCONATE 20% aqueous solution | 0.2 (0.04 of chlorhexidine gluconate) |
| SODIUM HYDRATE drops P.P.A | 0.0102 |
| MINT / LIQUORICE FLAVOURING | 0.1 |
| SODIUM SACCHARINE | 0.1 |
| | 100 |

### Method for preparation of the compositions of Examples 1-3

Kolliphor 407 is added to demineralised water and heated to 70° ± 2°C:
The mixture is cooled to 50°C ± 2°C under stirring, and a mixture of propylene glycol and chlorhexidine digluconate is then added, again under stirring. Xylitol is added at the same temperature, the mixture is cooled to 40° ± 2°C, and Cremophor RH40 is added at 40° ± 2°C. Finally, the flavourings and hexyl-resorcinol solution (Prawnfresh^{®}) are added. The flavourings and sodium saccharine are added to the resulting solution, the mixture is cooled to about 25°C, and the pH is adjusted to 5.20-5.80 by adding sodium hydroxide.

### Example 4 - Comparative tooth enamel pigmentation tests

The formulations of examples 1-3 were compared with similar ethylresorcinol-free formulations containing the same concentration of chlorhexidine (0.2%, 0.12% and 0.05%).

The tests were conducted by immersing in the test solutions tooth samples with uniform characteristics in terms of enamel condition, originating from tooth extractions. Each sample was subjected to 10 applications at 12-hour intervals with the test solutions, each application lasting 3 minutes.

The presence of chromophore on the surface of the tooth elements was evaluated visually after the treatment. The comparative test solutions of ethylresorcinol exhibited evident pigmentation after only two applications, which became even more evident after 10 applications, until a markedly brown pigmentation appeared. The samples treated with the formulations according to the invention did not exhibit any visually detectable colour change after two applications, and only slight pigmentation after 10 applications, but markedly less than that observed after only two applications with the comparator compositions.

## Claims

1. Sulphite-free compositions comprising chlorhexidine or a salt thereof and an alkyl-resorcinol in combination with suitable excipients.

2. Compositions according to claim 1 in the form of aqueous solutions.

3. Compositions according to claim 1 or 2 wherein chlorhexidine is in the form of a gluconate.

4. Compositions according to claim 1, 2 or 3 wherein the alkyl-resorcinol is 4-hexyl-resorcinol.

5. Compositions according to one or more of claims 1 to 4 wherein chlorhexidine, or a salt thereof, is present in concentrations ranging between 0.001 and 0.1% by weight.

6. Compositions according to claim 4 wherein chlorhexidine is present in concentrations ranging between 0.01 and 0.05% by weight.

7. Compositions according to one or more of claims 1 to 6 wherein 4-hexyl-resorcinol is present in concentrations ranging between 0.001 and 0.05% by weight.

8. Compositions according to claim 7 wherein 4-hexyl-resorcinol is present in concentrations ranging between 0.002 and 0.02% by weight.

9. Compositions according to one or more of claims 1 to 8 wherein the excipients comprise co-solvents; surfactants; flavourings/sweeteners; salts; pH adjusting agents; colouring agents; viscosity-regulating agents and mucoadhesive agents.

10. The compositions of claims 1 to 9 for use in disinfection of the oral cavity without pigmentation effects on the teeth.
